(19) Europäisches Patentamt / European Patent Office / Office européen des brevets

(11) EP 3 509 560 B1

(12) **EUROPEAN PATENT SPECIFICATION**

(45) Date of publication and mention of the grant of the patent:
**30.09.2020 Bulletin 2020/40**

(21) Application number: **17749178.4**

(22) Date of filing: **04.08.2017**

(51) Int Cl.:
***A61K 8/27*** (2006.01) ***A61K 8/46*** (2006.01)
***A61K 8/49*** (2006.01) ***A61Q 5/02*** (2006.01)
***A61K 8/73*** (2006.01) ***A61Q 17/00*** (2006.01)
***A61K 8/81*** (2006.01) ***A61Q 5/00*** (2006.01)

(86) International application number:
**PCT/EP2017/069793**

(87) International publication number:
**WO 2018/046206 (15.03.2018 Gazette 2018/11)**

(54) **ANTIMICROBIAL PERSONAL CLEANSING COMPOSITIONS**
ANTIMIKROBIELLE KÖRPERREINIGUNGSZUSAMMENSETZUNGEN
COMPOSITIONS D'HYGIÈNE CORPORELLE ANTIMICROBIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2016 PCT/CN2016/098315**

(43) Date of publication of application:
**17.07.2019 Bulletin 2019/29**

(73) Proprietors:
- **Unilever PLC**
  **London, Greater London EC4Y 0DY (GB)**
  Designated Contracting States:
  **CY GB IE MT**
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
- **DERICI, Leo**
  **Wirral**
  **Merseyside CH63 3JW (GB)**
- **CHANG, Shaokun**
  **Shanghai 200335 (CN)**
- **PI, Yingying**
  **Shanghai 200335 (CN)**

(74) Representative: **Chisem, Janet**
**Unilever Patent Group**
**Colworth House**
**Sharnbrook**
**Bedford MK44 1LQ (GB)**

(56) References cited:
**WO-A2-2011/131452** **US-A1- 2012 309 733**
**US-A1- 2014 154 200**

- **DATABASE GNPD [Online] MINTEL; 1 June 2013 (2013-06-01), "Anti-Dandruff Shampoo", XP002773661, Database accession no. 2086157**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the Invention

The present invention relates to antimicrobial personal cleansing compositions such as liquid soaps, body washes and shampoos.

Background of the Invention

In order to provide antimicrobial benefits in a cleansing base such as a liquid soap, body wash or shampoo, it has been proposed to include antimicrobial agents.

Zinc pyrithione (or ZPT) is an antimicrobial agent which is active against both gram-positive and gram-negative bacteria, as well as fungi and yeasts. It is widely used in antimicrobial personal cleansing compositions such as anti-dandruff (AD) shampoos. Generally, dispersed particles of the ZPT are suspended in the shampoo, which is then applied to the hair to deposit the ZPT particles on the hair and scalp.

WO 2011/131452 describes hair care compositions comprising high levels of pyrithione. The composition further comprises a polymer selected from acrylic acid based polymers and acrylate/C10-30 alkyl acrylate copolymers, and a water-soluble, nonionic cellulose.

US 2012/0309733 describes a hair treatment composition comprising a zinc-based antidandruff agent, a conazole fungicide and a cationic modified guar deposition polymer. It also relates to a method of enhancing deposition of zinc pyrithione in a shampoo composition.

US 2014/0154200 relates to a non-cellulosic polysaccharide derivative having a mean average molecular weight (Mw) from about 100,000g/mol, preferably from about 150,000g/mol and more preferably from about 200,000g/mole to about 2000000g/mol, preferably to about 1800000g/mol and more preferably to about 1400000g/mole; and containing at least one cationic group, with a cationic degree of subsitution $(DS_{cat})_{extraction}$, from about 0.20 to about 0.30.

The amount of AD active deposited onto the human scalp in the process of shampoo application and rinse-off is one of the key factors which determine the efficacy of an AD shampoo.

However, maximizing AD active deposition during cleansing is a difficult task since most personal cleansing compositions were designed to carry away particulates from the skin or hair.

Shampoo deposition of water insoluble actives like ZPT is most efficiently managed by using :a deposition agent such as a cationic deposition polymer. The polymer interacts with the surfactant system to form a complex which precipitates out of the shampoo upon use (dilution with water) and deposits the water insoluble active onto hair and scalp surfaces.

Efforts have also been made to increase the antimicrobial efficacy of ZPT by combining it with "booster" technologies. Certain zinc salts have been found to be effective as ZPT boosters in an AD shampoo context, although their mechanism of action is not fully understood.

However, the incorporation of additional zinc salts may impair product attributes such as flocculation behavior on dilution.

The present invention addresses this problem.

Summary of the Invention

The invention provides an antimicrobial personal cleansing composition comprising:

(i) an aqueous continuous phase including one or more anionic cleansing surfactants,

(ii) a dispersed phase including dispersed particles of zinc pyrithione (ZPT) in combination with one or more additional zinc salts;

(iii) a cationic deposition polymer selected from one or more cationic polygalactomannans having an average molecular weight ($M_w$) of from 1 million to 3 million g/mol and a cationic degree of substitution of from 0.13 to 0.3;

(iv) an anionic polymeric rheology modifier selected from one or more carboxylic acid polymers, and

(v) a nonionic polymeric rheology modifier selected from one or more nonionic cellulose ethers, wherein the cellulose ethers are (C1-3 alkyl) hydroxy (C1-3 alkyl) cellulose ethers.

Detailed Description and Preferred Embodiments

The antimicrobial personal cleansing composition according to the invention comprises an aqueous continuous phase (i) including one or more anionic cleansing surfactants.

By "aqueous continuous phase" is meant a continuous phase which has water as its basis. Suitably, the composition of the invention will comprise from about 50 to about 90%, preferably from about 55 to about 85%, more preferably from about 60 to about 85%, most preferably from about 65 to about 83% water (by weight based on the total weight of the composition).

Typical anionic cleansing surfactants for use in the invention include those surface active agents which contain an organic hydrophobic group with from 8 to 14 carbon atoms, preferably from 10 to 14 carbon atoms in their molecular structure; and at least one water-solubilising group which is preferably selected from sulphate, sulphonate, sarcosinate and isethionate.

Specific examples of such anionic cleansing surfactants include ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

A preferred class of anionic cleansing surfactants for use in the invention are alkyl ether sulphates of general formula:

$$R\text{-}O\text{-}(CH_2CH_2\text{-}O)_n\text{-}SO_3^-M^+$$

in which R is a straight or branched chain alkyl group having 10 to 14 carbon atoms, n is a number that represents the average degree of ethoxylation and ranges from 1 to 5, preferably from 1 to 3, and M is a alkali metal, ammonium or alkanolammonium cation, preferably sodium, potassium, monoethanolammonium or triethanolammonium, or a mixture thereof.

Specific examples of such preferred anionic cleansing surfactants include the sodium, potassium, ammonium or ethanolamine salts of $C_{10}$ to $C_{12}$ alkyl sulphates and $C_{10}$ to $C_{12}$ alkyl ether sulphates (for example sodium lauryl ether sulphate),

Mixtures of any of the above described materials may also be used.

In a typical composition according to the invention the level of anionic cleansing surfactant will generally range from 8 to 25%, and preferably ranges from 10 to 16% by weight based on the total weight of the composition.

In a preferred composition according to the invention the anionic cleansing surfactant is sodium lauryl ether sulphate (1EO), at a level of from 10 to 16% (by weight based on the total weight of the composition).

The aqueous continuous phase of the composition according to the invention preferably also includes one or more amphoteric surfactants, in addition to the anionic cleansing surfactant described above. Suitable amphoteric surfactants are betaines, such as those having the general formula $R(CH_3)_2N^+CH_2COO^-$, where R is an alkyl or alkylamidoalkyl group, the alkyl group preferably having 10 to 16 carbon atoms. Particularly suitable betaines are oleyl betaine, caprylamidopropyl betaine, lauramidopropyl betaine, isostearylamidopropyl betaine, and cocoamidopropyl betaine. Cocoamidopropyl betaine is particularly preferred.

When included, the total level of amphoteric surfactant is preferably from 0.1 to 10%, more preferably from 0.5 to 5%, and most preferably from 1 to 3% by weight based on the total weight of the hair cleansing composition).

The composition of the invention may suitably include at least one inorganic electrolyte. The inorganic electrolyte may be used to help provide viscosity to the composition. The term "inorganic electrolyte" in the context of this invention denotes an inorganic salt which dissolves in water and ionizes but whose ions do not aggregate in solution as, for example, do the ions of a surface active agent which aggregate to form micelles.

Suitable inorganic electrolytes for use in the invention include metal chlorides (such as sodium chloride, potassium chloride, calcium chloride, magnesium chloride, zinc chloride, ferric chloride and aluminium chloride) and metal sulphates (such as sodium sulphate and magnesium sulphate). The inorganic electrolyte is used to assist in the solubilisation of the hydrocarbon-based oily liquid conditioning agents (ii) and to provide viscosity to the composition.

Examples of preferred inorganic electrolytes for use in the invention include sodium chloride, potassium chloride, magnesium sulphate and mixtures thereof.

Mixtures of any of the above described materials may also be suitable.

The composition of the invention may suitably have a viscosity ranging from 3,000 to 10,000 mPa.s, preferably

from 4,000 to 9,000 mPa.s when measured using a Brookfield V2 viscometer (spindle RTV5, 1 minute, 20rpm) at 30°C.

The antimicrobial personal cleansing composition according to the invention comprises a dispersed phase (ii) including dispersed particles of zinc pyrithione (ZPT) in combination with one or more additional zinc salts.

Zinc pyrithione (ZPT) has the following chemical structure:

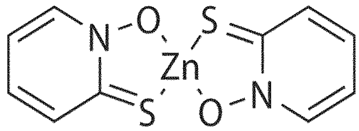

The ZPT particles may be amorphous, or may take various regular or irregular crystalline forms such as rods, needles, blocks, platelets and mixtures thereof. The average particle diameter of the ZPT particles (maximum dimension) is typically from about 0.1 to about 50 μm, preferably from about 0.1 m to about 10 μm, more preferably from about 0.1 μm to about 5 μm as determined, for example, using a Horiba LA-910 Laser scattering particle size distribution analyzer.

The level of ZPT in compositions of the invention generally ranges from about 0.1 to about 3%, and preferably ranges from about 0.2 to about 2%, more preferably from about 0.5 to about 1.5%, by weight based on the total weight of the composition.

The one or more additional zinc salts may suitably be selected from zinc salts of organic acids, zinc salts of inorganic acids, zinc oxides, zinc hydroxides and mixtures thereof.

Examples of additional zinc salts for use in the invention include zinc oxide, zinc pyrrolidone carboxylic acid, zinc citrate, zinc carbonate, zinc chloride, zinc sulphate, zinc glycinate, zinc acetate, zinc lactate, and mixtures thereof.

Additional zinc salts for use in the invention preferably have a zinc mass% of at least 25%, more preferably at least 30% (based on total mass of the zinc salt).

Additional zinc salts for use in the invention preferably have a solubility in water of 20g/l or less, more preferably 0.1g/l or less at 25°C.

Examples of preferred additional zinc salts for use in the invention include zinc oxide, zinc pyrrolidone carboxylic acid, zinc citrate, zinc carbonate and mixtures thereof.

The level of additional zinc salt(s) in compositions of the invention generally ranges from about 0.1 to about 5%, and preferably ranges from about 0.2 to about 3%, more preferably from about 0.25 to about 2.5%, by weight based on the total weight of the composition.

In a particularly preferred composition according to the invention the additional zinc salt is selected from zinc oxide, zinc pyrrolidone carboxylic acid, zinc citrate, zinc carbonate and mixtures thereof; at a level ranging from about 0.25 to about 2.5% by weight based on the total weight of the composition.

The antimicrobial personal cleansing composition according to the invention comprises a cationic deposition polymer (iii) which is selected from one or more cationic polygalactomannans having an average molecular weight ($M_w$) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans for use as the cationic deposition polymer (iii) in the invention may be selected from guar and hydroxyalkyl guar (for example hydroxyethyl guar or hydroxypropyl guar), that has been cationically modified by chemical reaction with one or more derivatizing agents.

Derivatizing agents typically contain a reactive functional group, such as an epoxy group, a halide group, an ester group, an anhydride group or an ethylenically unsaturated group, and at least one cationic group such as a cationic nitrogen group, more typically a quaternary ammonium group. The derivatization reaction typically introduces lateral cationic groups on the polygalactomannan backbone, generally linked via ether bonds in which the oxygen atom corresponds to hydroxyl groups on the polygalactomannan backbone which have reacted.

Preferred cationic polygalactomannans for use as the cationic deposition polymer (iii) in the invention include guar hydroxypropyltrimethylammonium chlorides.

Guar hydroxypropyltrimethylammonium chlorides are generally comprised of a nonionic guar backbone that is

functionalized with ether-linked 2-hydroxypropyltrimethylammonium chloride groups, and are typically prepared by the reaction of guar with 3-chloro-2-hydroxypropyl) trimethylammonium chloride or 2,3-epoxypropyl trimethylammonium chloride.

The term "cationic degree of substitution" (DS) in the context of the present invention refers to the average substitution of cationic functional groups per glycoside unit in the polygalactomannan molecule. In guar, on average each of the glycoside units contains three available hydroxyl sites. A DS of three would mean that all of the available hydroxyl sites have been esterified with cationic functional groups. Average DS values can be expressed as decimal fractions of these integer values, and mean that the polygalactomannan molecule comprises glycoside units having whole number DS values embracing the average. DS values may suitably be measured using $^1$H-NMR spectroscopy after hydrolysis in a $DCl/D_2O$ mixture (as described for example in Bigand et al. Carbohydrate Polymers 85 (2011) 138-148). The DS can be directly determined from the relative integration of protons of the cationic functional groups (e.g. hydroxypropyltrimethylammonium groups) to the integration of the anomeric protons corresponding to $\alpha$ and $\beta$ conformations of galactose and mannose.

The cationicity of the cationic polygalactomannans for use as the cationic deposition polymer (iii) in the invention may also be expressed in terms of cationic charge density. The term "cationic charge density" in the context of the present invention refers to the ratio of positive charges on a monomeric unit of which a polymer is comprised to the molecular weight of said monomeric unit. The charge density multiplied by the polymer molecular weight determines the number of positively charged sites on a given polymer chain. The cationic charge density of guar hydroxypropyltrimethylammonium chlorides may be calculated from the DS using the following equation:

$$\text{Cationic charge density in milliequivalents per gram (meq/g)} = \frac{DS \times 1000}{162 + 151 \times DS}$$

In general, the equation above depends on the cationic group which is grafted to the polygalactomannan backbone.

Preferred cationic polygalactomannans for use as the cationic deposition polymer (iii) in the invention have a DS of from 0.16 to 0.3. This DS value corresponds to a charge density of from 0.85 to 1.45, calculated using the above equation.

The term "average molecular weight ($M_w$)" in the context of the present invention refers to the weight average molecular weight. The average molecular weight ($M_w$) may be is determined by SEC (Size Exclusion Chromatography) analysis using an ELSD (Evaporative Light Scattering Detector). The average molecular weight ($M_w$) is calculated using a calibration curve generated with a set of pullulan standards.

Preferred cationic polygalactomannans for use as the cationic deposition polymer (iii) in the invention have an average molecular weight ($M_w$) of from 1 million to 2.5 million g/mol.

One class of preferred cationic polygalactomannan for use as the cationic deposition polymer (iii) in the invention includes guar hydroxypropyltrimethylammonium chlorides having a DS of from 0.16 to 0.20 and an average molecular weight ($M_w$) of from 1 million to 1.5 million g/mol. A specific example of such a material is guar hydroxypropyltrimethylammonium chloride having a DS of 0.18 and an average molecular weight ($M_w$) of about 1.35 million g/mol, from Lamberti S.p.A.

Another class of preferred cationic polygalactomannan for use as the cationic deposition polymer (iii) in the invention includes guar hydroxypropyltrimethylammonium chlorides having a DS of from 0.25 to 0.30 and an average molecular weight ($M_w$) of from 2 million to 2.5 million g/mol. A specific example of such a material is Jaguar®C17, from Solvay.

Mixtures of any of the above described materials may also be used.

In a typical composition according to the invention the level of cationic polygalactomannans will generally range from about 0.05 to about 1%, and preferably ranges from 0.1 to 0.8%, more preferably from 0.2 to 0.6% by weight based on the total weight of the composition.

In a particularly preferred composition according to the invention the cationic polygalactomannan is one or more guar hydroxypropyltrimethylammonium chlorides having a DS of from 0.16 to 0.30 and an average molecular weight ($M_w$) of from 1 million to 2.5 million g/mol; at a level ranging from 0.2 to 0.6% by weight based on the total weight of the composition.

The antimicrobial personal cleansing composition according to the invention comprises an anionic polymeric rheology modifier (iv) selected from carboxylic acid polymers.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include α-β-unsaturated monocarboxylic acids such as acrylic acid, methacrylic acid and crotonic acid; and α-β-unsaturated dicarboxylic acids such as itaconic acid, fumaric acid, maleic acid and aconitic acid. Salts, esters or anhydrides of the α-β-unsaturated mono- or dicarboxylic acids described above may also be used. Examples include half esters of α-β-unsaturated dicarboxylic acids with $C_{1-4}$ alkanols, such as monomethyl fumarate; cyclic anhydrides of α-β-unsaturated dicarboxylic acids such as maleic anhydride, itaconic anhydride and citraconic anhydride; and esters of acrylic acid or methacrylic acid with $C_{1-30}$ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof.

Carboxylic acid polymers for use as the anionic polymeric rheology modifier (iv) in the invention preferably have a molecular weight of at least 1 million g/mol.

Carboxylic acid polymers for use as the anionic polymeric rheology modifier (iv) in the invention may suitably be crosslinked.

Typical crosslinking monomers include polyalkenyl polyethers having at least two polymerizable ethylenically unsaturated double bonds. The term "polyalkenyl polyether" in the context of this invention refers to alkenyl ethers of organic polyols wherein the organic polyol is etherified by reacting it with an alkenyl halide, such as allyl chloride or allyl bromide. The polyalkenyl polyether (e.g. polyallyl polyether) can contain 2 to 8 polymerizable ethylenically unsaturated double bonds. Suitable polyols for the etherification reaction can contain 2 to 12 carbon atoms and have at least two hydroxyl groups. The polyol can be linear, branched or cyclic (e.g. monosaccharides and polysaccharides containing 1 to 4 saccharide units). Specific examples of suitable polyalkenyl polyethers include polyallyl ethers of sucrose having from 2 to 8 allyl groups per molecule, pentaerythritol diallyl ether, pentaerythritol triallyl ether, pentaerythritol tetraallyl ether; trimethylolpropane diallyl ether, trimethylolpropane triallyl ether, and mixtures thereof.

A suitable carboxylic acid polymer for use as the anionic polymeric rheology modifier (iv) in the invention is a crosslinked homopolymer polymerized from acrylic acid or methacrylic acid (typically crosslinked with an allyl ether of pentaerythritol, or an allyl ether of sucrose). Such materials may generally be referred to under the INCI name of Carbomer. Commercially available examples include Carbopol® polymers 934, 940 and 980 from Lubrizol Advanced Materials.

Also suitable are crosslinked copolymers polymerized from $C_{1-4}$ alkyl acrylate or methacrylate (e.g. ethyl acrylate) with one or more comonomers selected from acrylic acid, methacrylic acid and mixtures thereof. Such materials may generally be referred to under the INCI name of Acrylates Copolymer. Commercially available examples include Aculyn® 33 from Rohm and Haas.

Also suitable are crosslinked copolymers polymerized from $C_{10-30}$ alkyl esters of acrylic or methacrylic acid with one or more comonomers selected from acrylic acid, methacrylic acid and their respective $C_{1-4}$ alkyl esters. Such materials may generally be referred to under the INCI name of Acrylates/C10-30 Alkyl Acrylate Crosspolymer. Commercially available examples include Carbopol® polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCI names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn® 22, 28 or 88 from Rohm & Haas and Synthalen® from 3V Sigma.

Carboxylic acid polymers for use as the anionic polymeric rheology modifier (iv) in the invention are preferably selected from Carbomers, such as homopolymers of acrylic acid crosslinked with an allyl ether of pentaerythritol or an allyl ether of sucrose.

Mixtures of any of the above described materials may also be used.

In a typical composition according to the invention the level of carboxylic acid polymers will generally range from about 0.1 to about 1%, and preferably ranges from 0.4 to 0.8% by weight based on the total weight of the composition.

In a particularly preferred composition according to the invention the carboxylic acid polymer is one or more homopolymers of acrylic acid crosslinked with an allyl ether of pentaerythritol or an allyl ether of sucrose; at a level ranging from 0.4 to 0.8% by weight based on the total weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The pH of the final, fully-formulated composition of the invention preferably ranges from 4 to 7, more preferably from 5.5 to 6.5.

The antimicrobial personal cleansing composition according to the invention comprises a nonionic polymeric

rheology modifier (v) which is selected from one or more nonionic cellulose ethers.

Suitable nonionic cellulose ethers or use as the nonionic polymeric rheology modifier (v) in the invention include ($C_{1-3}$ alkyl) cellulose ethers, such as methyl cellulose and ethyl cellulose; hydroxy ($C_{1-3}$ alkyl) cellulose ethers, such as hydroxyethyl cellulose and hydroxypropyl cellulose; mixed hydroxy ($C_{1-3}$ alkyl) cellulose ethers, such as hydroxyethyl hydroxypropyl cellulose; and ($C_{1-3}$ alkyl) hydroxy ($C_{1-3}$ alkyl) cellulose ethers, such as hydroxyethyl methylcellulose and hydroxypropyl methylcellulose.

Preferred nonionic cellulose ethers for use as the nonionic polymeric rheology modifier (v) in the invention are water-soluble nonionic cellulose ethers such as methylcellulose and hydroxypropyl methylcellulose. The term "water-soluble" in this context denotes a solubility in water of at least 1 grams, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL® trademark series. Generally, these materials are manufactured by heating cellulose fibres with caustic solution which in turn is treated with methyl chloride to obtain methoxyl substitution on the anhydroglucose units. Methylcellulose is made using only methyl chloride. For hydroxypropyl methylcellulose, propylene oxide is used in addition to methyl chloride to obtain hydroxypropoxyl substitution on the anhydroglucose units. The amount of substituent groups on the anhydroglucose units influences the solubility properties of the cellulose ether. Methylcelluloses and hydroxypropyl methylcelluloses for use as the nonionic polymeric rheology modifier (v) in the invention generally have a sufficient degree of methoxyl or methoxyl/hydroxypropoxyl substitution to cause them to be water-soluble as defined above.

Examples of preferred nonionic cellulose ethers for use as the nonionic polymeric rheology modifier (v) in the invention include hydroxypropyl methylcelluloses having a methoxyl substitution of from about 10% to about 40%, more preferably from about 15% to about 30%, and a hydroxypropoxyl substitution of from about 1% to about 15%, more preferably from about 2% to about 10%. All the percentages of substitution are by weight of the finally substituted material. Methoxyl and hydroxypropoxyl substitution may be measured and calculated according to ASTM D2363-79 (2011).

Preferred nonionic cellulose ethers for use as the nonionic polymeric rheology modifier (v) in the invention, such as the hydroxypropyl methylcelluloses described above, can have a viscosity ranging from about 1,500 mPa.s to about 25,000 mPa.s, more preferably from about 3,000 mPa.s to about 15,000 m.Pas, when measured in a 2wt% aqueous solution at 20°C using an Ubbelohde viscometer according to ASTM D2363-79 (2011).

A commercially available example of a preferred nonionic cellulose ether for use as the nonionic polymeric rheology modifier (v) in the invention is METHOCEL® 40-202 from Dow Chemical (a hydroxypropyl methylcellulose having a methoxyl content of 28-30%, a hydroxypropoxyl content of 7-12%, and a viscosity of about 4,000 mPa.s).

Mixtures of any of the above nonionic cellulose ethers may also be suitable.

In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

In a particularly preferred composition according to the invention the nonionic cellulose ether is a water-soluble hydroxypropyl methylcellulose (such as is further described above); at a level ranging from 0.1 to 0.3% by weight based on the total weight of the composition.

The composition of the invention may also include emulsified droplets of non-volatile silicone having a mean droplet diameter (D3,2) of 1 micrometre or less. Preferably the mean droplet diameter (D3,2) is 1 micrometre or less, more preferably 0.5 micrometre or less, and most preferably 0.25 micrometre or less.

A suitable method for measuring the mean droplet diameter (D3,2) is by laser light scattering using an instrument such as a Malvern Mastersizer.

The term "non-volatile silicone" in the context of this invention means a silicone with a vapour pressure of less than 1000 Pa at 25°C.

Suitable silicones for use in the invention include polydiorganosiloxanes, in particular polydimethylsiloxanes (dimethicones), polydimethyl siloxanes having hydroxyl end groups (dimethiconols), and amino-functional polydimethylsiloxanes (amodimethicones).

Suitable silicones preferably have a molecular weight of greater than 100,000 and more preferably a molecular weight of greater than 250,000.

All molecular weights as used herein are weight average molecular weights, unless otherwise specified.

Suitable silicones preferably have a kinematic viscosity of greater than 50,000 cS

($mm^2.s^{-1}$) and more preferably a kinematic viscosity of greater than 500,000 cS ($mm^2.s^{-1}$). Silicone kinematic viscosities in the context of this invention are measured at 25°C and can be measured by means of a glass capillary viscometer as set out further in Dow Corning Corporate Test Method CTM004 July 20, 1970.

Suitable silicones for use in the invention are available as pre-formed silicone emulsions from suppliers such as Dow Corning and GE Silicones. The use of such pre-formed silicone emulsions is preferred for ease of processing

and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Pre-formed silicone emulsions having a mean droplet diameter (D3,2) of less than 0.15 micrometres are generally termed microemulsions.

Examples of suitable pre-formed silicone emulsions include emulsions DC2-1766, DC2-1784, DC-1785, DC-1786, DC-1788, DC-1310, DC-7123, DC5-7128 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Mixtures of any of the above described silicone emulsions may also be used.

When included, the amount of emulsified, non-volatile silicone in compositions of the invention may suitably range from 0.05 to 10%, preferably from 0.2 to 8% (by total weight silicone based on the total weight of the composition).

A composition of the invention may contain further optional ingredients to enhance performance and/or consumer acceptability. Examples of such ingredients include fragrance, dyes and pigments and preservatives. Each of these ingredients will be present in an amount effective to accomplish its purpose. Generally these optional ingredients are included individually at a level of up to 5% by weight based on the total weight of the composition.

Mode of Use

The composition of the invention is primarily intended for topical application to the body, preferably the hair and scalp.

Most preferably the composition of the invention is topically applied to the hair and then massaged into the hair and scalp. The composition is then rinsed off the hair and scalp with water prior to drying the hair.

The invention will be further illustrated by the following, non-limiting Examples, in which all percentages quoted are by weight based on total weight unless otherwise stated.

## EXAMPLES

A hair cleansing shampoo formulation was prepared, having ingredients as shown in Table 1 below. Example 1 represents a formulation according to the invention.

Table 1

| Ingredient | Example 1 |
|---|---|
| Sodium laureth sulphate (1EO) | 14 |
| Cocamidopropyl betaine | 1.6 |
| Carbopol®980 (ex Lubrizol) | 0.6 |
| METHOCEL® 40-202 (ex Dow Chemical) | 0.2 |
| Zinc pyrithione (ZPT) | 1 |
| Zinc sulphate hexahydrate | 0.1 |
| Zinc oxide | 1 |
| Sodium hydroxide | 0.3 |
| Citric acid | 1.2 |
| Cationic guar* | 0.2 |
| DOW CORNING® 5-7128 silicone emulsion | 0.8 |
| DOW CORNING® 1788 silicone emulsion | 1.2 |
| Sodium benzoate | 0.3 |
| Disodium EDTA | 0.5 |
| Sodium chloride | 0.35 |
| Water, minors | q.s.to 100 |
| ** Guar hydroxypropyltrimethylammonium chloride having a DS of 0.18 and an average molecular weight ($M_w$) of about 1.35 million g/mol, from Lamberti S.p.A.* | |

Figure 1 shows a series of micrographs of the shampoo of Example 1, compared to a control formulation omitting the METHOCEL® 40-202, each after dilution x10 with water.

The micrographs of the diluted Example 1 all show fine, uniformly dispersed particles of ZPT. By contrast, the micrographs of the diluted control show an irregular distribution of ZPT particles, and a number of areas where particles appear to have clustered together to form flocculates.

## Claims

1. An antimicrobial personal cleansing composition comprising:

   (i) an aqueous continuous phase including one or more anionic cleansing surfactants,
   (ii) a dispersed phase including dispersed particles of zinc pyrithione (ZPT) in combination with one or more additional zinc salts;
   (iii) a cationic deposition polymer selected from one or more cationic polygalactomannans having an average molecular weight ($M_w$) of from 1 million to 3 million g/mol and a cationic degree of substitution of from 0.13 to 0.3;
   (iv) an anionic polymeric rheology modifier selected from one or more carboxylic acid polymers, and
   (v) a nonionic polymeric rheology modifier selected from one or more nonionic cellulose ethers, wherein the cellulose ethers are (C1-3 alkyl) hydroxy (C1-3 alkyl) cellulose ethers.

2. A composition according to claim 1, in which the anionic cleansing surfactant is sodium lauryl ether sulphate (1EO), at a level of from 10 to 16% (by weight based on the total weight of the composition).

3. A composition according to claim 1 or claim 2, in which the additional zinc salt is selected from zinc oxide, zinc pyrrolidone carboxylic acid, zinc citrate, zinc carbonate and mixtures thereof; at a level ranging from 0.25 to 2.5% by weight based on the total weight of the composition.

4. A composition according to any one of claims 1 to 3, in which the cationic polygalactomannan is one or more guar hydroxypropyltrimethylammonium chlorides having a DS of from 0.16 to 0.30 and an average molecular weight ($M_w$) of from 1 million to 2.5 million g/mol; at a level ranging from 0.2 to 0.6% by weight based on the total weight of the composition.

5. A composition according to any one of claims 1 to 4, in which the carboxylic acid polymer is one or more homopolymers of acrylic acid crosslinked with an allyl ether of pentaerythritol or an allyl ether of sucrose; at a level ranging from 0.4 to 0.8% by weight based on the total weight of the composition.

6. A composition according to any one of claims 1 to 5, in which the nonionic cellulose ether is a water-soluble hydroxypropyl methylcellulose; at a level ranging from 0.1 to 0.3% by weight based on the total weight of the composition.

7. A composition according to claim 6, in which the hydroxypropyl methylcellulose has a methoxyl substitution of from 15% to 30%, and a hydroxypropoxyl substitution of from 2% to 10% (by weight of the finally substituted material).

## Patentansprüche

1. Antimikrobielle Körperreinigungszusammensetzung, umfassend:

   (i) eine wässrige kontinuierliche Phase, die ein oder mehrere anionische Reinigungstenside enthält,
   (ii) eine disperse Phase, die dispergierte Partikel von Zinkpyrithion (ZPT) in Kombination mit einem oder mehreren zusätzlichen Zinksalzen enthält;
   (iii) ein kationisches Abscheidungspolymer, das aus einem oder mehreren kationischen Polygalactomannanen mit einem durchschnittlichen Molekulargewicht ($M_w$) von 1 Million bis 3 Millionen g/mol und einem kationischen Substitutionsgrad von 0,13 bis 0,3 ausgewählt ist;
   (iv) einen anionischen polymeren Rheologiemodifikator, der aus einem oder mehreren Carbonsäurepolymeren ausgewählt ist, und
   (v) einen nichtionischen polymeren Rheologiemodifikator, der aus einem oder mehreren nichtionischen Celluloseethern ausgewählt ist, wobei die Celluloseether (C1-3-Alkyl)-hydroxy-(C1-3-alkyl)-Celluloseether sind.

2. Zusammensetzung nach Anspruch 1, bei der das anionische Reinigungstensid Natriumlaurylethersulfat (1 EO) in einer Menge von 10 bis 16% (nach Gewicht, bezogen auf das Gesamtgewicht der Zusammensetzung) ist.

3. Zusammensetzung nach Anspruch 1 oder Anspruch 2, wobei das zusätzliche Zinksalz ausgewählt ist aus Zinkoxid, Zinkpyrrolidoncarbonsäure, Zinkcitrat, Zinkcarbonat und Gemischen davon; in einer Menge im Bereich von 0,25 bis 2,5 Gewichts-%, bezogen auf das Gesamtgewicht der Zusammensetzung.

4. Zusammensetzung nach irgendeinem der Ansprüche 1 bis 3, wobei das kationische Polygalactomannan ein oder mehrere Guarhydroxypropyltrimethylammoniumchloride mit einem DS von 0,16 bis 0,30 und einem durchschnittlichen Molekulargewicht ($M_w$) von 1 Million bis 2,5 Millionen g/mol ist; in einer Menge im Bereich von 0,2 bis 0,6 Gewichts-%, bezogen auf das Gesamtgewicht der Zusammensetzung.

5. Zusammensetzung nach irgendeinem der Ansprüche 1 bis 4, wobei das Carbonsäurepolymer ein oder mehrere Homopolymere von Acrylsäure ist, die mit einem Allylether von Pentaerythrit oder einem Allylether von Saccharose vernetzt sind; in einer Menge im Bereich von 0,4 bis 0,8 Gewichts-%, bezogen auf das Gesamtgewicht der Zusammensetzung.

6. Zusammensetzung nach irgendeinem der Ansprüche 1 bis 5, wobei der nichtionische Celluloseether eine wasserlösliche Hydroxypropylmethylcellulose ist; in einer Menge im Bereich von 0,1 bis 0,3 Gewichts-%, bezogen auf das Gesamtgewicht der Zusammensetzung.

7. Zusammensetzung nach Anspruch 6, wobei die Hydroxypropylmethylcellulose eine Methoxylsubstitution von 15% bis 30% und eine Hydroxypropoxylsubstitution von 2% bis 10% (bezogen auf das Gewicht des fertig substituierten Materials) aufweist.

**Revendications**

1. Composition nettoyante antimicrobienne pour la personne comprenant :

   (i) une phase continue aqueuse incluant un ou plusieurs tensioactifs nettoyants anioniques,
   (ii) une phase dispersée incluant des particules dispersées de zinc pyrithione (ZPT) en combinaison avec un ou plusieurs sels de zinc supplémentaires ;
   (iii) un polymère de dépôt cationique choisi parmi un ou plusieurs polygalactomannanes cationiques ayant une masse moléculaire moyenne (*Mw*) de 1 million à 3 millions g/mol et un degré cationique de substitution de 0,13 à 0,3 ;
   (iv) un agent de modification de rhéologie polymère anionique choisi parmi un ou plusieurs polymères d'acide carboxylique, et
   (v) un agent de modification de rhéologie polymère non ionique choisi parmi un ou plusieurs éthers de cellulose non ioniques, dans laquelle les éthers de cellulose sont des éthers de (alkyle en C1-3) hydroxy (alkyle en C1-3) cellulose.

2. Composition selon la revendication 1, dans laquelle le tensioactif de nettoyant anionique est le lauryléthersulfate de sodium (1EO) ; à une teneur de 10 à 16 % (en masse sur la base de la masse totale de la composition).

3. Composition selon la revendication 1 ou la revendication 2, dans laquelle le sel de zinc supplémentaire est choisi parmi l'oxyde de zinc, l'acide zinc pyrrolidone carboxylique, le citrate de zinc, le carbonate de zinc et des mélanges de ceux-ci ; à une teneur de 0,25 à 2,5 % en masse sur la base de la masse totale de la composition.

4. Composition selon l'une quelconque des revendications 1 à 3, dans laquelle le polygalactomannane cationique est un ou plusieurs chlorures de guar hydroxypropyltriméthylammonium présentant un DS de 0,16 à 0,30 et une masse moléculaire moyenne ($M_w$) de 1 million à 2,5 millions g/mol ; à une teneur de 0,2 à 0,6 % en masse sur la base de la masse totale de la composition.

5. Composition selon l'une quelconque des revendications 1 à 4, dans laquelle le polymère d'acide carboxylique est un ou plusieurs homopolymères d'acide acrylique réticulés avec un allyléther de pentaérythritol ou un allyléther de saccharose ; à une teneur de 0,4 à 0,8 % en masse sur la base de la masse totale de la composition.

6. Composition selon l'une quelconque des revendications 1 à 5, dans laquelle l'éther de cellulose non ionique est une hydroxypropyl-méthylcellulose soluble dans l'eau ; à une teneur de 0,1 à 0,3 % en masse sur la base de la masse totale de la composition.

7. Composition selon la revendication 6, dans laquelle l'hydroxypropylméthylcellulose présente une substitution méthoxyle de 15 % à 30 %, et une substitution hydroxypropoxyle de 2 % à 10 % (en masse du matériau finalement substitué).

Fig. 1

Example 1 (x10 dilution)

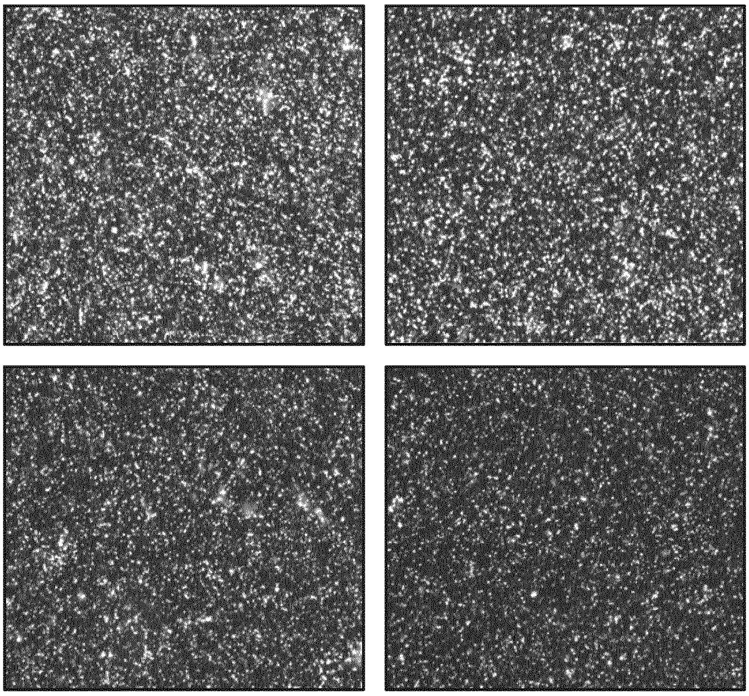

Fig. 2

Control (x10 dilution)

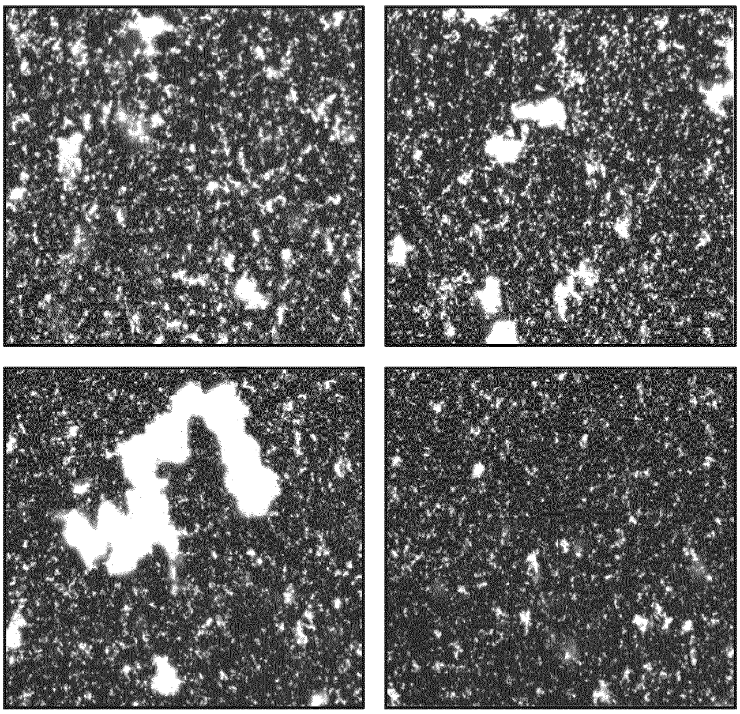

SUBSTITUTE SHEET (RULE 26)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011131452 A
- US 20120309733 A
- US 20140154200 A


### Non-patent literature cited in the description


- **BIGAND et al.** *Carbohydrate Polymers,* 2011, vol. 85, 138-148